# FASCICULE DE BREVET EUROPEEN

(11) **EP 1 814 455 B1**
(45) Date de publication et mention de la délivrance du brevet: **17.08.2011**
(21) Numéro de dépôt: 05778091.8
(22) Date de dépôt: 24.06.2005
(51) Int. Cl.: A61B 5/103

(54) **DISPOSITIF DE PREVENTION D'ESCARRE**
VORRICHTUNG ZUR VERHINDERUNG VON SCHORF
ESCHAR PREVENTION DEVICE

(30) Priorité: 24.06.2004 FR 0451319
(43) Date de publication de la demande: 08.08.2007
(73) Titulaire: Centre National de la Recherche Scientifique, 75794 Paris Cedex 16 (FR)
(72) Inventeur: PAYAN, Yohan, F-38580 ALLEVARD (FR); DEMONGEOT, Jacques, F-38360 SASSENAGE (FR); VAZQUEZ-BUENOSAIRES, Jose-Octavio, TUXTLA GUTIERREZ, 29000 (MX)
(74) Mandataire: de Beaumont, Michel
(86) Numéro de dépôt international: PCT/FR2005/050490
(87) Numéro de publication internationale: WO 2006/008406

(56) Documents cités:
- US-A- 4 554 930
- US-A- 6 030 351

## Description

### Domaine de l'invention

La présente invention concerne des équipements de fauteuils roulants de personnes handicapées moteurs, para ou tétraplégiques ainsi que des lits de personnes handicapées ou malades. Plus particulièrement, la présente invention concerne un dispositif de prévention d'escarre.

### Exposé de l'art antérieur

En position assise ou couchée, un individu non handicapé ne cesse d'être animé de mouvements réflexes, le plus souvent imperceptibles, pour mouvoir des zones d'appui de cet individu ce qui permet d'éviter la formation d'escarres. Par contre, une personne blessée médullaire ou handicapée moteur para ou tétraplégique, immobilisée partiellement ou complètement en position assise ou couchée n'a pas de tels mouvements réflexe.

Pour les personnes blessées médullaires ou handicapées moteur para ou tétraplégiques, on a proposé un dispositif d'apprentissage clinique. Ce dispositif est utilisé en milieu hospitalier pour une personne ayant déjà présenté des escarres. La personne est placée sur une assise constituée d'un siège d'une dimension de 43x43 cm dont la surface est équipée d'une matrice de 30x30 capteurs de pression. Les capteurs de pression sont régulièrement répartis à la surface du siège. Les capteurs fournissent alors une carte de couleur de niveau de pression. Cette carte est alors observée en temps réel par le patient et par un spécialiste qui explique à la personne assise comment modifier sa position pour prendre une position idéale. Dans une telle position idéale, qui dépend essentiellement de la morphologie et/ou du handicap de la personne, les pressions sont sensiblement équi-réparties. Les risques d'escarres sont alors considérablement réduits, voire nuls.

Un tel dispositif présente de nombreux inconvénients, tout d'abord, il n'est la plupart du temps mis en oeuvre qu'après l'apparition d'escarre alors que la résorption de ce type de nécrose est particulièrement longue et douloureuse chez une personne ne pouvant se tenir debout. Ensuite il requiert le déplacement de la personne assise dans un centre hospitalier ce qui est parfois difficilement compatible avec des activités professionnelles. Par ailleurs, on a observé que si les patients surveillent leur position dans leur fauteuil habituel dans les quelques heures à quelques jours qui suivent leur venue au centre, ils cessent très vite de surveiller leur position et reprennent leurs mauvaises positions habituelles conduisant à la formation de nouvelles escarres. De plus, l'efficacité d'un tel apprentissage périodique semble inversement proportionnel à l'âge du patient.

La présente invention vise par conséquent à proposer un dispositif de prévention d'escarres qui soit plus simple à mettre en oeuvre.

La présente invention vise à proposer un tel dispositif qui soit embarqué dans le fauteuil d'une personne handicapée moteur.

La présente invention vise également à proposer un tel dispositif qui s'adapte à la morphologie d'un patient donné.

### Résumé de l'invention

Pour atteindre ces objets, la présente invention prévoit un dispositif de prévention de formation d'escarres au niveau d'une zone d'appui d'une personne assise ou couchée, comprenant :
des capteurs de pression sous la zone d'appui ;
des moyens de comparaison de chaque mesure fournie par un quelconque des capteurs à au moins une valeur seuil ;
des moyens de mémorisation propres à mémoriser toutes les mesures fournies par tous les capteurs pendant une première durée à partir d'un dépassement de la valeur seuil par au moins une mesure ;
des moyens de traitement des mesures mémorisées propres à déterminer, une fois la première durée écoulée, des instructions de déplacement de la personne assise ou couchée pour éviter la formation d'escarres ;
un dispositif d'électro-stimulation linguale propre à transmettre les instructions de déplacement à la personne assise ou couchée ; et
des actionneurs actionnables par la personne assise ou couchée et propres à provoquer son déplacement.

Selon un mode de réalisation de la présente invention, le dispositif comprend en outre :
des moyens pour déterminer après un dépassement de la valeur seuil pour un capteur donné si les mesures fournies ultérieurement par le capteur donné deviennent inférieures à la valeur seuil ;
des moyens pour mesurer le temps pendant lequel les mesures fournies par le capteur demeurent inférieures à la valeur seuil ; et
des moyens pour interrompre pour le capteur donné tout traitement de mémorisation des mesures et/ou de transmission d'instructions pour ce capteur si et dès que le temps atteint une seconde durée prédéterminée.

Selon un mode de réalisation de la présente invention, la valeur de la première durée dépend de l'écart à la valeur seuil de la mesure ayant dépassé la valeur seuil.

Selon un mode de réalisation de la présente invention, la valeur seuil est égale à 13,332.10³ Pa (100 mm Hg) et la première durée est égale à trente minutes si la valeur mesurée est inférieure à 15,332.10³ Pa (115 mm Hg), la première durée est égale à quinze minutes si la valeur mesurée est comprise entre 15,332.10³ Pa (115 mm Hg) et 17,331.10³ Pa (130 mm Hg), et la première durée est égale à dix minutes si la valeur mesurée est supérieure à 17,331.10³ Pa (130 mm Hg).

Selon un mode de réalisation de la présente invention, les capteurs de pression sont répartis sous la zone d'appui à l'intersection des lignes et colonnes d'une matrice à douze lignes et douze colonnes subdivisée en deux sous-matrices à douze lignes et six colonnes, l'écart entre deux lignes successives de chaque sous-matrice étant, depuis l'arrière vers l'avant de la zone d'appui, 2 cm, 1,5 cm, 1,5 cm, 1 cm, 1 cm, 1 cm, 1,5 cm, 1,5 cm, 2 cm, 3 cm et 4 cm, et l'écart entre deux colonnes successives de chaque sous-matrice étant 3,5 cm, 3 cm, 2 cm, 3 cm et 3,5 cm.

Selon un mode de réalisation de la présente invention, les deux sous-matrices sont adjacentes.

Selon un mode de réalisation de la présente invention, les deux sous-matrices sont disjointes.

Selon un mode de réalisation de la présente invention, l'écart entre les deux sous-matrices est réglable sur la zone d'appui en fonction de la morphologie de la personne assise ou couchée.

Selon un mode de réalisation de la présente invention, le dispositif d'électro-stimulation linguale est constitué d'une matrice carrée de trente-six électrodes.

Selon un mode de réalisation de la présente invention, les moyens de traitement des mesures sont propres à calculer des moyennes des mesures mémorisées pendant la première durée, à déterminer et à ordonner parmi les moyennes obtenues les maxima dépassant la valeur seuil et à déterminer des instructions de déplacement de la personne assise ou couchée pour supprimer les maxima.

### Brève description des dessins

Ces objets, caractéristiques et avantages, ainsi que d'autres de la présente invention seront exposés en détail dans la description suivante de modes de réalisation particuliers faite à titre non-limitatif en relation avec les figures jointes parmi lesquelles :
la figure 1 illustre schématiquement les zones à risque de formation des escarres ;
la figure 2 illustre une matrice de capteurs utilisée dans un dispositif selon la présente invention ;
la figure 3 illustre un premier codage de correction de position selon la présente invention ; et
la figure 4 illustre un autre codage de correction de position selon la présente invention.

### Description détaillée

Par souci de clarté, de mêmes éléments ont été désignés par de mêmes références aux différentes figures. De plus, les figures ne sont pas tracées à l'échelle.

La présente invention sera décrite ci-après dans le cas d'une personne assise. Toutefois, l'homme de l'art comprendra que le dispositif selon la présente invention peut également être utilisé pour une personne alitée.

La présente invention tire parti des observations des inventeurs sur l'existence de zones particulièrement sensibles pour la formation d'escarre au niveau de la zone d'appui d'une personne assise.

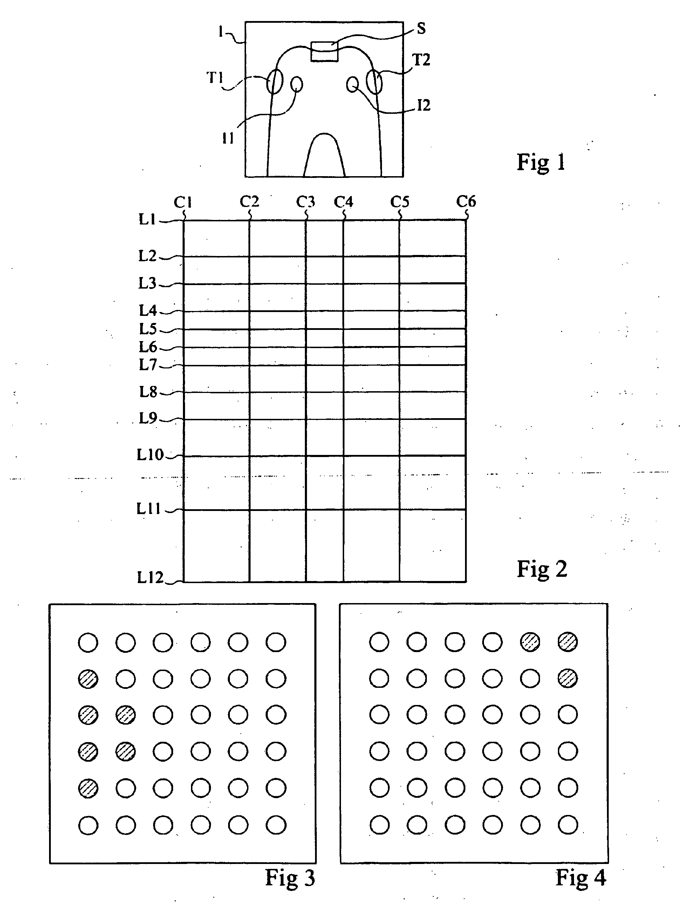

La figure 1 illustre schématiquement un siège 1 d'un fauteuil sur lequel une personne est assise. Cette personne présente cinq zones sensibles réparties symétriquement en trois zones. Ces zones sont les zones d'appui I1 et I2 des ischions gauche et droit, les zones d'appui T1 et T2 des trochanters gauche et droit et la zone d'appui centrale S du sacrum.

Le dispositif selon la présente invention comporte une matrice de 12x12 capteurs constituée de deux sous-matrices à douze lignes et six colonnes. La matrice de capteurs est placée sur le siège d'un fauteuil dans la partie arrière de la zone d'assise d'une dimension standard de 43x43 cm et a une dimension totale de 30x30 cm. Chaque sous-matrice présente une même répartition irrégulière des capteurs choisie de façon à concentrer un plus grand nombre de capteurs dans les zones sensibles.

La figure 2 illustre schématiquement une telle sous-matrice. Depuis l'arrière vers l'avant, les douze lignes sont numérotées L1 à L12. Les colonnes sont désignées de gauche à droite par C1, C2, C3, C4, C5 et C6. L'écart séparant deux lignes successives varie de la façon suivante :
L1 - L2 : 2 cm ;
L2 - L3 : 1,5 cm ;
L3 - L4 : 1,5 cm ;
L4 - L5 : 1 cm ;
L5 - L6 : 1 cm ;
L6 - L7 : 1 cm ;
L7 - L8 : 1,5 cm ;
L8 - L9 : 1,5 cm ;
L9 - L10 _{:} 2 cm ;
L10 - L11 : 3 cm ;
L11 - L12 _{:} 4 cm.

L'écart entre deux colonnes successives est le suivant .
C1 - C2 _{:} 3,5 cm ;
C2 - C3 : 3 cm ;
C3 - C4 _{:} 2 cm ;
C4 - C5 _{:} 3 cm ;
C5 - C6 : 3,5 cm.

Selon un mode de réalisation de la présente invention, les deux sous-matrices sont solidaires.

Selon un mode de réalisation de la présente invention, les deux sous-matrices sont disjointes, munies de dispositifs amovibles d'accrochage au siège. Cela permet de s'adapter à la morphologie de la personne assise en modifiant l'écart entre les sous-matrices de façon qu'elles soient chacune centrée sur un ischion.

Les 12x12 capteurs Sij répartis à l'intersection des lignes Lᵢ et colonnes Cj fournissent des mesures de pression Mᵢⱼ au niveau de la zone d'appui de la personne assise. Les mesures Mᵢⱼ sont effectuées toutes les secondes, c'est-à-dire avec une fréquence de 1 Hz.

Le dispositif selon l'invention comporte des moyens propres à recevoir les mesures Mᵢⱼ et à les comparer à au moins une valeur seuil. Par exemple, les mesures Mᵢⱼ sont comparées à trois valeurs seuil successives croissantes. Par exemple, les valeurs seuil sont des pressions de 13,332.10³ Pa, 15,332.10³ Pa et 17,331.10³ Pa (100, 115 et 130 millimètres de mercure).

Le dispositif selon l'invention comporte des moyens de mémorisation. Ces moyens de mémorisation sont activés dès qu'une mesure Mᵢⱼ dépasse l'une des valeurs seuil, et mémorisent alors chaque groupe de mesures Mᵢⱼ fournies par tous les capteurs Sᵢⱼ. A l'intérieur de chaque groupe (matrice) de mesures Mij, chaque mesure Mᵢⱼ occupe un emplacement correspondant à l'emplacement du capteur Sᵢⱼ l'ayant fournie.

Les groupes de 144 mesures instantanées Mᵢⱼ sont mémorisés pendant une durée qui dépend de l'écart de la mesure ayant provoqué la mémorisation par rapport à la valeur seuil. Dans le cas considéré où plusieurs valeurs seuils sont fixées, la durée de mémorisation dépend de la valeur seuil initialement dépassée. Plus la valeur seuil est élevée, plus la durée de mémorisation est courte. Par exemple, lorsque la mesure Mᵢⱼ ayant déclenché la mémorisation est supérieure à 17,331.10³ Pa (130 mm Hg), la durée de la mémorisation est de dix minutes. Lorsque la mesure Mᵢⱼ est comprise entre 15,332.10³ et 17, 331.10³ Pa (115 et 130 mm Hg), la durée de la mémorisation est de 15 minutes. Lorsque la mesure Mᵢⱼ est comprise entre 13,332.10³ et 15,332.10³ Pa (100 et 115 mm Hg), la durée est de 30 minutes.

Le dispositif selon la présente invention comporte des moyens propres à sélectionner la durée de mémorisation à partir du résultat de la comparaison et des moyens propres à déterminer l'écoulement de cette durée.

Le dispositif selon la présente invention comporte également des moyens propres à traiter, une fois la durée de mémorisation écoulée, tous les groupes de mesures mémorisés pendant cette durée. Plus particulièrement, de tels moyens de traitement comportent des moyens propres à calculer pour chaque capteur Sᵢⱼ la valeur moyenne de pression pendant la durée de mémorisation. Les valeurs moyennes sont mémorisées dans un groupe (matrice) de même dimension que les groupes de mesures instantanées Mᵢⱼ. Les moyens de traitement comportent en outre des moyens de comparaison pour déterminer dans l'ensemble des valeurs moyennes l'existence de maxima supérieurs à la valeur seuil la plus basse de 13,332.10³ Pa (100 mm Hg). Les moyens de traitement comportent de plus des moyens propres à ordonner les différents maxima par ordre croissant.

Le dispositif selon l'invention comporte un moyen de type table de vérité propre à associer à une valeur moyenne un capteur donné Sᵢⱼ. Une fois déterminé le maximum le plus élevé, un tel moyen détermine à quel capteur il est associé. Le dispositif comporte des moyens propres à déterminer la position morphologique d'un capteur et des moyens propres à déterminer à partir d'une telle position une instruction de modification de position propre à faire disparaître le maximum.

L'instruction de modification de position est une instruction de déplacement précise telle que vers la gauche, vers la droite, vers l'avant, vers l'arrière, vers l'avant gauche ou droite ou encore vers l'arrière gauche ou droite. Les instructions de modification de position permettent de revenir ou de prendre une position sensiblement idéale dans laquelle les surpressions sont supprimées.

L'instruction de modification de position est transmise au patient par l'intermédiaire d'un dispositif buccal appelé dispositif d'électrosimulation linguale ou dispositif TDU (tongue display unit).

Dans le dispositif selon la présente invention, le dispositif d'électro-stimulation linguale est un dispositif fixé au palais du patient et comportant un plateau à seulement 6x6 électrodes. Chaque possibilité de déplacement du patient est associée à la stimulation d'un groupe prédéfini d'électrodes.

Par exemple, la figure 3 illustre schématiquement la matrice de carrée 6x6 électrodes. Pour indiquer au patient qu'il doit se déplacer vers la gauche, six électrodes sont stimulées, à savoir les quatre électrodes centrales de la colonne de gauche et les deux électrodes centrales de la colonne voisine. Les six électrodes stimulées sont hachurées en figure 3.

En figure 3, la partie basse est la partie fixée dans la cavité palatine contre les dents supérieures et la partie haute la partie du dispositif électro-stimulation linguale avançant vers l'intérieur de la cavité. Un déplacement vers l'avant sera donc indiqué de préférence par une activation des électrodes de la partie basse de la figure 3 et vers l'arrière par une activation des électrodes de la partie haute de la figure 3.

Ainsi, pour indiquer un déplacement vers l'arrière et vers la droite, les trois électrodes formant un angle dans la partie inférieure droite du dispositif électro-stimulation linguale lorsque celui-ci est fixé au palais de la personne assise sont activées. Comme l'illustre schématiquement la figure 4, les électrodes activées sont alors les trois électrodes hachurées formant un angle entre la première ligne et la dernière colonne de la matrice d'électrodes.

Sur la base de l'instruction de déplacement ainsi reçue, la personne assise peut se déplacer de façon correspondante.

Le dispositif selon l'invention comporte des moyens de contrôle pour vérifier la disparition du maximum ayant provoqué l'instruction. Les moyens de contrôle comportent des moyens de comparaison propre à comparer la mesure associée au capteur ayant provoqué l'instruction à la valeur seuil la plus basse de 13,332.10³ Pa (100 mm Hg). Les moyens de contrôle mesurent le temps écoulé à partir de la disparition d'un maximum. Dès que le maximum a disparu depuis une durée de remise à zéro déterminée comprise entre une et deux minutes, les moyens de contrôle provoquent l'interruption de la transmission de l'instruction de déplacement. On notera que si lors de ce contrôle, la mesure fournie passe à nouveau au-dessus de la valeur seuil, la mesure de la durée de remise à zéro est interrompue et que l'instruction continue à être fournie à la personne assise.

Dans le cas où plusieurs maxima ont été détectés, l'homme de l'art comprendra qu'il est fortement probable que ces maxima sont morphologiquement rapprochés, situés dans une même zone sensible. Un déplacement propre à faire disparaître le maximum le plus élevé peut par conséquent faire disparaître plusieurs maxima. Les moyens de contrôle surveillent donc en parallèle tous les maxima précédemment déterminés et mesurent le cas échéant plusieurs durées de remise à zéro.

Après l'écoulement de la durée de remise à zéro pour le maximum pour lequel une instruction de déplacement était fournie à la personne assise, les moyens de contrôle déterminent si le maximum moins élevé suivant est toujours détecté. Un maximum est considéré comme toujours détecté si la mesure fournie par le capteur correspondant est toujours supérieure à la valeur seuil la plus basse ou est inférieure à cette valeur seuil depuis moins que la durée de remise à zéro.

Si un maximum est toujours détecté, alors le moyen de traitement fournit à la personne assise, par l'intermédiaire du dispositif électro-stimulation linguale, l'instruction de déplacement requise.

Pour une personne paraplégique, les instructions de déplacement peuvent souvent être prises en compte directement et la personne se déplace elle-même de la façon appropriée.

Selon un mode de réalisation, la présente invention comporte également des actionneurs propres à être actionnés par la personne assise ou alitée qui permettent à celle-ci de modifier sa position.

On a indiqué précédemment que les moyens de mémorisation commencent à mémoriser toutes les mesures des capteurs dès que la mesure d'un capteur a dépassé un seuil puis que des moyens de contrôle interviennent lors de la transmission d'instruction de déplacement. Toutefois, de préférence, les moyens de contrôle sont activés dès qu'une mémorisation commence pour un capteur donné et interrompent la mémorisation pour ce capteur si la valeur qu'il fournit devient et demeure inférieure à la valeur seuil la plus basse pendant la durée de remise à zéro. En effet, la durée de remise à zéro nécessaire pour garantir qu'une escarre est non susceptible de se former à l'emplacement surveillé par le capteur est extrêmement courte par rapport à la durée pendant laquelle doit se maintenir une surpression pour qu'une escarre soit susceptible de se former. Il n'est pas nécessaire de continuer la mémorisation alors que le risque d'escarre a déjà disparu, ce qui serait de toute façon révélé par l'absence de maximum pour le capteur considéré au bout de la durée de mémorisation.

Un tel dispositif présente de nombreux avantages.

Tout d'abord, il peut facilement être placé et laissé en place dans un fauteuil ou un lit d'un patient car il est peu encombrant. En effet, le nombre de capteurs est réduit de 900 à 144 soit de plus d'un facteur 6. La connectique associée est donc relativement réduite et peu encombrante. De plus, le dispositif selon la présente invention n'utilise pas d'affichage visuel. Cela réduit considérablement l'encombrement par rapport au dispositif de test clinique connu.

En outre, le dispositif électro-stimulation linguale utilisé présente des dimensions réduites. En effet, les dispositifs TDU couramment utilisés dans d'autres applications présentent des dimensions de l'ordre de 4x4 cm propre à recevoir 12x12 électrodes. Le dispositif selon la présente invention utilise une matrice de 6x6 électrodes de sensiblement deux centimètres de côté.

## Revendications

1. Dispositif de prévention de formation d'escarres au niveau d'une zone d'appui d'une personne assise ou couchée, comprenant :
des capteurs de pression sous la zone d'appui ;
des moyens de comparaison de chaque mesure fournie par un quelconque des capteurs à au moins une valeur seuil ;
des moyens de mémorisation propres à mémoriser toutes les mesures fournies par tous les capteurs pendant une première durée à partir d'un dépassement de la valeur seuil par au moins une mesure, le dispositif étant **caractérisé par**
des moyens de traitement des mesures mémorisées propres à déterminer, une fois la première durée écoulée, des instructions de déplacement de la personne assise ou couchée pour éviter la formation d'escarres ;
un dispositif d'électro-stimulation linguale propre à transmettre les instructions de déplacement à la personne assise ou couchée ; et
des actionneurs actionnables par la personne assise ou couchée et propres à provoquer son déplacement.

2. Dispositif selon la revendication 1, comprenant en outre :
des moyens pour déterminer après un dépassement de la valeur seuil pour un capteur donné si les mesures fournies ultérieurement par ledit capteur donné deviennent inférieures à la valeur seuil ;
des moyens pour mesurer le temps pendant lequel les mesures fournies par ledit capteur demeurent inférieures à la valeur seuil ; et
des moyens pour interrompre pour le capteur donné tout traitement de mémorisation des mesures et/ou de transmission d'instructions pour ce capteur si et dès que ledit temps atteint une seconde durée prédéterminée.

3. Dispositif selon la revendication 1 ou 2, dans lequel la valeur de la première durée dépend de l'écart à la valeur seuil de la mesure ayant dépassé ladite valeur seuil.

4. Dispositif selon la revendication 3, dans lequel la valeur seuil est égale à 13, 332.10³ Pa (100 mm Hg) et en ce que la première durée est égale à trente minutes si la valeur mesurée est inférieure à 15,332.10³ Pa (115 mm Hg), la première durée est égale à quinze minutes si la valeur mesurée est comprise entre 15,332.10³ Pa (115 mm Hg) et 17,331.10³ Pa (130 mm Hg), et en ce que la première durée est égale à dix minutes si la valeur mesurée est supérieure à 17,331.10³ Pa (130 mm Hg).

5. Dispositif selon l'une quelconque des revendications 1 à 4, dans lequel les capteurs de pression sont répartis sous la zone d'appui à l'intersection des lignes (L1, L2, L3, L4, L5, L6, L7, L8, L9, L10, L11, L12) et colonnes (C1, C2, C3, C4, C5, C6) d'une matrice à douze lignes et douze colonnes subdivisée en deux sous-matrices à douze lignes et six colonnes, l'écart entre deux lignes successives de chaque sous-matrice étant, depuis l'arrière vers l'avant de la zone d'appui, 2 cm, 1,5 cm, 1,5 cm, 1 cm, 1 cm, 1 cm, 1,5 cm, 1,5 cm, 2 cm, 3 cm et 4 cm, et l'écart entre deux colonnes successives de chaque sous-matrice étant 3,5 cm, 3 cm, 2 cm, 3 cm et 3,5 cm.

6. Dispositif selon la revendication 5, dans lequel les deux sous-matrices sont adjacentes.

7. Dispositif selon la revendication 5, dans lequel les deux sous-matrices sont disjointes.

8. Dispositif selon la revendication 5, dans lequel l'écart entre les deux sous-matrices est réglable sur la zone d'appui en fonction de la morphologie de la personne assise ou couchée.

9. Dispositif selon l'une quelconque des revendications 1 à 8, dans lequel le dispositif d'électro-stimulation linguale est constitué d'une matrice carrée de trente-six électrodes.

10. Dispositif selon l'une quelconque des revendications 1 à 9, dans lequel les moyens de traitement des mesures sont propres à calculer des moyennes des mesures mémorisées pendant la première durée, à déterminer et à ordonner parmi les moyennes obtenues les maxima dépassant la valeur seuil et à déterminer des instructions de déplacement de la personne assise ou couchée pour supprimer lesdits maxima.

## Claims

1. A device for preventing the forming of eschars at the level of a seated or lying person's bearing area, comprising:
pressure sensors under the bearing area;
means for comparing each measurement provided by any one of the sensors with at least one threshold value;
memorization means capable of memorizing all the measurements provided by all the sensors for a first time period from an exceeding of the threshold value by at least one measurement;
the device being **characterized by**:
means for processing the memorized measurements capable of determining, once the first time period has elapsed, displacement instructions for the seated or lying person to avoid the forming of eschars;
a tongue display unit capable of transmitting the displacement instruction to the seated or lying person; and
actuators operable by the seated or lying person and capable of causing his or her displacement.

2. The device of claim 1, further comprising:
means for determining after an exceeding of the threshold value for a given sensor whether the measurements subsequently provided by said given sensor become lower than the threshold value;
means for measuring the time for which the measurements provided by said sensor remain lower than the threshold value; and
means for interrupting for the given sensor any processing of memorization of the measurements and/or of transmission of instructions for this sensor if and as soon as the time reaches a second predetermined time period.

3. The device of claim 1 or 2, in which the value of the first time period depends on the distance from the threshold value of the measurement having exceeded said threshold value.

4. The device of claim 3, in which the threshold value is equal to 13.332.10³ Pa (100 mm Hg) and in which the first time period is equal to thirty minutes if the measured value is lower than 15.332.10³ Pa (115 mm Hg), the first time period is equal to fifteen minutes if the measured value ranges between 15.332.10³ Pa (115 mm Hg) and 17.331.10³ Pa (130 mm Hg), and in which the first time period is equal to ten minutes if the measured value is greater than 17.331.10³ Pa (130 mm Hg).

5. The device of any of claims 1 to 4, in which the pressure sensors are distributed under the bearing area at the intersection of the lines (L1, L2, L3, L4, L5, L6, L7, L8, L9, L10, L11, L12) and columns (C1, C2, C3, C4, C5, C6) of a matrix with twelve lines and twelve columns divided into two sub-matrixes with twelve lines and six columns, the interval between two successive lines of each sub-matrix being, from the rear to the front of the bearing area, 2 cm, 1.5 cm, 1.5 cm, 1 cm, 1 cm, 1 cm, 1.5 cm, 1.5 cm, 2 cm, 3 cm, and 4 cm, and the interval between two successive columns of each sub-matrix being 3.5 cm, 3 cm, 2 cm, 3 cm, and 3.5 cm.

6. The device of claim 5, in which the two sub-matrixes are adjacent.

7. The device of claim 5, in which the two sub-matrixes are separate.

8. The device of claim 5, in which the interval between the two sub-matrixes is settable on the bearing area according to the seated or lying person's morphology.

9. The device of any of claims 1 to 8, in which the tongue display unit is formed of a square matrix of thirty-six electrodes.

10. The device of any of claims 1 to 9, in which the means for processing the measurements are capable of calculating averages of the measurements memorized for the first time period, of determining and of ordering among the obtained averages the maximum values exceeding the threshold value and of determining instructions for displacing the seated or lying person to suppress said maximum values.

## Patentansprüche

1. Vorrichtung zur Verhinderung der Schorfbildung im Bereich einer Auflagefläche einer sitzenden oder liegenden Person, welche Folgendes aufweist:
Drucksensoren unter der Auflagefläche;
Mittel zum Vergleichen jeder Messung, die von einem der Sensoren geliefert wird, mit mindestens einem Schwellenwert;
Speichermittel, die geeignet sind, alle Messungen, die von allen Sensoren geliefert werden, zu speichern, und zwar für eine erste Zeitperiode ab einem Übertreten des Schwellenwerts von mindestens einer Messung;
wobei die Vorrichtung **gekennzeichnet ist durch**:
Mittel zum Verarbeiten der gespeicherten Messungen, die geeignet sind, sobald die erste Zeitperiode verstrichen ist, Verlagerungs- bzw. Umlagerungsanweisungen für die sitzende oder liegende Person zu bestimmen, um die Schorfbildung zu vermeiden;
eine Einheit zur Elektrostimulation der Zunge bzw. eine Zungen-Display-Einheit (Tongue-Display-Unit), die geeignet ist, um die Umlagerungsanweisungen an die sitzende oder liegende Person zu übermitteln; und
Betätigungsvorrichtungen, die von der sitzenden oder liegenden Person betätigbar sind, und die geeignet sind, ihre Ver- bzw. Umlagerung herbeizuführen.

2. Vorrichtung nach Anspruch 1, welche ferner Folgendes aufweist:
Mittel, um nach einem Überschreiten des Schwellenwertes für einen bestimmten Sensor zu bestimmen, ob die Messungen, die im Folgenden von dem bestimmten Sensor geliefert werden, niedriger als der Schwellenwert werden;
Mittel, um die Zeit zu messen, in welcher die von dem vorgegebenen Sensor gelieferten Messungen niedriger bleiben als der Schwellenwert; und
Mittel, um für den bestimmten Sensor jede Speicherung, Messungen und/oder Übermittlung der Anweisungen für diesen Sensor zu unterbrechen, wenn und sobald die Zeit eine zweite vorbestimmte Zeitperiode erreicht.

3. Vorrichtung nach Anspruch 1 oder 2, in der der Wert der ersten Zeitperiode von dem Abstand von dem Schwellenwert der Messung abhängt, die den Schwellenwert überschritten hat.

4. Vorrichtung nach Anspruch 3, in der der Schwellenwert gleich 13.332.10³ Pa (100mm Hg) ist und in der die erste Zeitperiode gleich dreißig Minuten ist, wenn der gemessene Wert niedriger als 15.332.10³ Pa (115mm Hg) ist, die erste Zeitperiode gleich fünfzehn Minuten ist, wenn der gemessene Wert in einer Größenordnung zwischen 15.332.10³ Pa (115mm Hg) und 17.331.10³ Pa (130mm Hg) liegt und in der die erste Zeitperiode gleich 10 Minuten ist, wenn der gemessene Wert größer als 17.331.10³ Pa (130mm Hg) ist.

5. Vorrichtung nach einem der Ansprüche 1 bis 4, in der die Drucksensoren unter der Auflagefläche an den Schnittpunkten der Zeilen (L1, L2, L3, L4, L5, L6, L7, L8, L9, L10, L11, L12) und Spalten (C1, C2, C3, C4, C5, C6) einer Matrix mit 12 Zeilen und 12 Spalten verteilt sind, die in zwei Submatrices mit 12 Zeilen und 6 Spalten geteilt ist, wobei das Intervall zwischen zwei aufeinanderfolgenden Zeilen jeder Submatrix von der Hinterseite zur Vorderseite der Auflagefläche 2 cm, 1,5 cm, 1,5 cm, 1 cm, 1 cm, 1 cm, 1,5 cm, 1,5 cm, 2 cm, 3 cm und 4 cm beträgt, und das Intervall zwischen zwei aufeinanderfolgenden Spalten jeder Submatrix 3,5 cm, 3 cm, 2 cm, 3 cm und 3,5 cm beträgt.

6. Vorrichtung nach Anspruch 5, in welcher die zwei Submatrices unmittelbar nebeneinander liegen.

7. Vorrichtung nach Anspruch 5, in der die zwei Submatrices getrennt sind.

8. Vorrichtung nach Anspruch 5, in der das Intervall zwischen den zwei Submatrices an der Auflagefläche gemäß der Morphologie der sitzenden oder liegenden Person einstellbar ist.

9. Vorrichtung nach einem der Ansprüche 1 bis 8, in welcher die Zungen-Display-Einheit aus einer quadratischen Matrix von sechsunddreißig Elektroden gebildet ist.

10. Vorrichtung nach einem der Ansprüche 1 bis 9, in welcher die Mittel zum Verarbeiten der Messungen geeignet sind, um Durchschnitte der Messungen, die für die erste Zeitperiode gespeichert wurden, zu berechnen, um unter den erhaltenen Durchschnitten die Maximalwerte, die den Schwellenwert überschreiten, zu bestimmen und zu ordnen, und um die Anweisungen zur Umlagerung der sitzenden oder liegenden Person zu bestimmen, um die Maximalwerte zu unterdrücken.
